# FASCICULE DE BREVET EUROPEEN

(11) **EP 3 125 972 B1**
(45) Date de publication et mention de la délivrance du brevet: **03.12.2025**
(21) Numéro de dépôt: 14721915.8
(22) Date de dépôt: 02.04.2014
(51) Int. Cl.: A61M 5/00, A61M 5/315

(54) **ENSEMBLE DE SERINGUE ANTI-REFLUX**
ANTIREFLUX-SPRITZENANORDNUNG
ANTI-REFLUX SYRINGE SET

(43) Date de publication de la demande: 08.02.2017
(73) Titulaire: Aptar Stelmi SAS, 93420 Villepinte (FR)
(72) Inventeur: SWAL, Mickaël, 77124 Chauconin Neufmontiers (FR); FOURNIER, Ghislain, 17000 La Rochelle (FR)
(74) Mandataire: CAPRI
(86) Numéro de dépôt international: PCT/FR2014/050790
(87) Numéro de publication internationale: WO 2015/150646

(56) Documents cités:
- WO-A1-2006/074171
- US-A- 3 331 538
- US-A1- 2004 010 235
- US-A1- 2005 154 353

## Description

La présente invention concerne un ensemble de seringue anti-reflux.

Les ensembles de seringue anti-reflux sont connus dans l'état de la technique, notamment des documents WO2004033006, WO2005032628, WO2008151239, WO2008151241, WO2011126764, US2004010235, US2005154353, US3331538 et WO2006074171. Ces dispositifs présentent certains inconvénients. Ainsi, pour éviter le collage entre deux ou plusieurs pistons lors de la phase de stockage, avant assemblage des pistons dans les réservoirs, on prévoit généralement des profils saillants, tels que des nervures, sur la face d'extrémité proximale des pistons, qui est la face en contact avec le produit fluide. Ces profils saillants empêchent l'effet ventouse qui peut se créer lorsque la face d'extrémité proximale d'un piston vient coopérer de manière étanche avec l'extrémité distale d'un autre piston. Par contre, la présence de profils saillants sur la face d'extrémité proximale du piston empêche soit de minimiser le volume mort qui subsiste entre le piston et le réservoir après actionnement, soit de garantir la fermeture étanche de la sortie du réservoir par le piston après actionnement et donc la fonction anti-reflux.

La présente invention a pour but de fournir un ensemble de seringue anti-reflux qui ne reproduit pas les inconvénients susmentionnés.

En particulier, la présente invention a pour but de fournir un ensemble de seringue anti-reflux qui soit fiable d'utilisation et qui permet de minimiser le volume mort qui subsiste entre le piston et le réservoir après actionnement.

La présente invention a aussi pour but de fournir des ensembles de seringue anti-reflux pour lesquels les risques de collage des pistons entre eux pendant la phase de stockage desdits pistons, avant assemblage dans lesdits ensembles, sont limités voire supprimés.

Plus particulièrement, la présente invention a pour but de fournir un ensemble de seringue anti-reflux qui soit simple et peu coûteux à fabriquer, à stocker et à assembler.

La présente invention a donc pour objet un ensemble de seringue anti-reflux selon la revendication 1. Des modes de réalisation avantageux sont décrits dans les revendications dépendantes.

Ces caractéristiques et avantages et d'autres de la présente invention apparaitront plus clairement au cours de la description détaillée suivante d'un mode de réalisation avantageux de celle-ci, faite en référence aux dessins joints, donnés à titre d'exemple non limitatifs, et sur lesquels
La figure 1 est une vue schématique découpée d'un ensemble de seringue selon un mode de réalisation avantageux de la présente invention, avant actionnement,
La figure 2 est une vue similaire à celle de la figure 1, après actionnement,
La figure 3a est une vue schématique de côté d'un piston selon un mode de réalisation avantageux de la présente invention,
La figure 3b est une vue en section transversale du piston de la figure 3a,
La figure 4a est une vue en perspective de devant du piston des figures 3a et 3b,
La figure 4b est une vue similaire à celle de la figure 4a, vu de derrière,
La figure 5 est une schématique en section transversale d'une partie d'un ensemble de seringue selon un mode de réalisation avantageux de la présente invention, avant utilisation,
La figure 6 est une vue de détail en section transversale de l'ensemble de seringue de la figure 5, après utilisation, et
Les figures 7 et 8 sont des vues schématiques de deux pistons coopérant l'un avec l'autre lors du stockage, avant assemblage dans un ensemble de seringue.

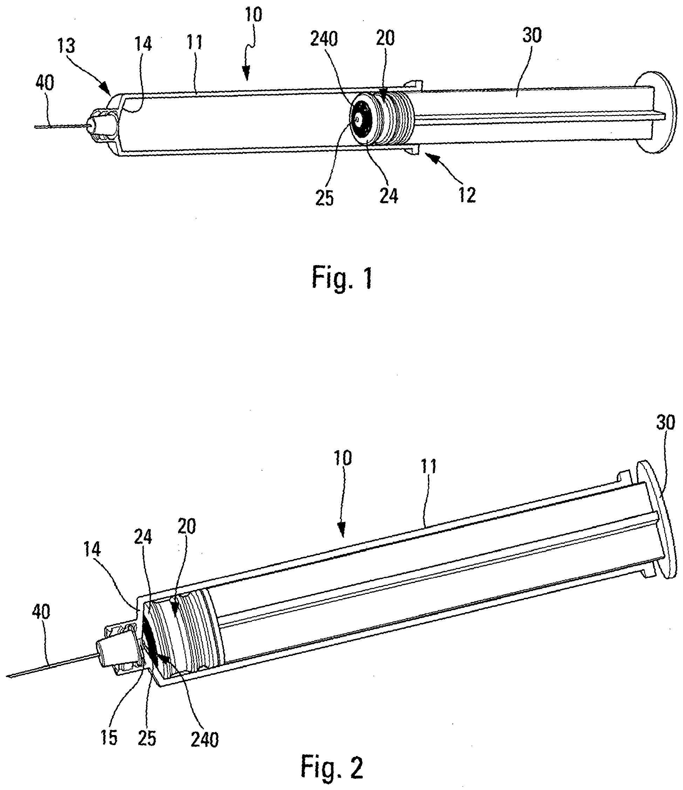

En référence aux figures 1, 2, 5 et 6, il est représenté un ensemble de seringue anti-reflux selon un mode de réalisation avantageux de l'invention.

Cet ensemble de seringue comporte un réservoir 10 destiné à contenir un produit fluide, un piston 20 coulissant de manière étanche dans ledit réservoir 10 en étant connecté à une tige de piston 30, pour expulser le produit fluide contenu dans ledit réservoir à travers une aiguille 40 fixée sur l'extrémité proximale 13 du réservoir 10.

Ledit réservoir comporte une paroi latérale cylindrique 11, une extrémité distale 12 ouverte et une extrémité proximale 13 comportant une paroi d'extrémité proximale 14 pourvue en son centre axial d'une ouverture de passage 15. L'aiguille 40 se fixe d'une quelconque manière appropriée sur ladite extrémité proximale 13 du réservoir pour se connecter à ladite ouverture de passage 15.

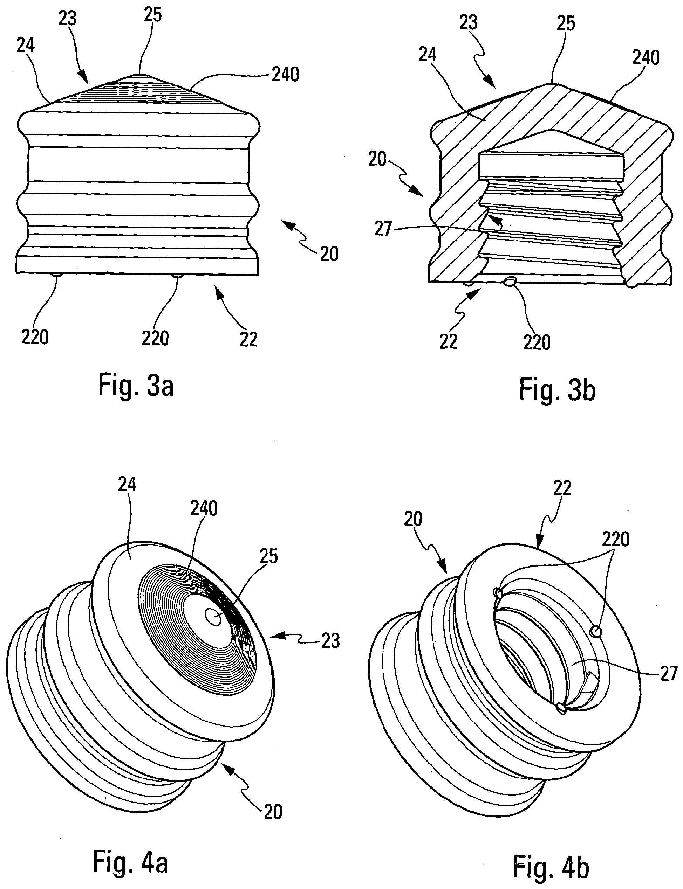

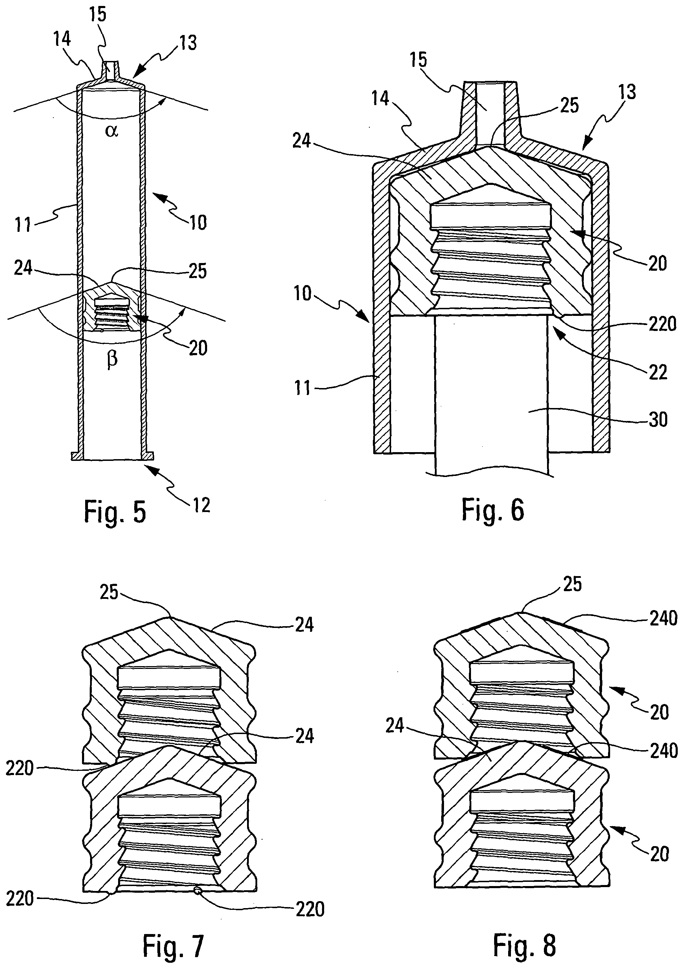

Ledit piston 20, représenté en détail sur les figures 3a, 3b, 4a et 4b, comporte une extrémité distale 22 et une extrémité proximale 23 comportant une paroi d'extrémité proximale 24. Ladite paroi d'extrémité proximale 24 du piston comporte une pointe axiale 25 adaptée à coopérer de manière étanche, après actionnement, avec ladite ouverture de passage 15 du réservoir 10, comme visible sur la figure 6.

Ladite extrémité distale ouverte 12 du réservoir 10 est destinée à recevoir ladite tige de piston 30. Ladite tige de piston 30 est adaptée à se connecter à ladite extrémité distale 22 du piston 20, avantageusement au moyen d'un pas de vis externe (non représenté) formé sur ladite tige de piston 30 et qui coopère avec un pas de vis interne 27 formé dans ledit piston 20. Le piston 20 est ainsi avantageusement vissé sur ladite tige de piston 30. D'autres moyens de fixation du piston 20 sur la tige de piston 30 sont aussi envisageables.

Selon l'invention, lesdites parois d'extrémités proximales 14, 24 du réservoir 10 et du piston 20 sont coniques. Ladite paroi d'extrémité proximale 14 du réservoir 10 forme un angle α et ladite paroi d'extrémité proximale 24 du piston 20 forme un angle β. Selon un aspect de l'invention, la différence entre l'angle α et l'angle β est comprise entre 3° et 5°, de préférence est égale à 4°. Le fait que cette différence soit supérieur ou égale à 3° garantit que la pointe 25 du piston vient effectivement fermer de manière étanche l'ouverture de passage 15 après actionnement, sans risque de reflux. Le fait que cette différence soit inférieure ou égale à 5°, de préférence égale à 4°, permet de limiter le volume mort qui reste entre le piston et le réservoir après actionnement, et garantit donc l'expulsion de la quasi-totalité du produit fluide contenu avant actionnement dans ledit réservoir 10.

Selon un mode de réalisation préféré, l'angle α est égal à 145° (+/- 1°) et l'angle β est égal à 141° (+/- 1°).

Selon un aspect avantageux de la présente invention, le piston 20 comporte des moyens anti-collage pour éviter le collage de deux ou plusieurs pistons 20 entre eux lors de la phase de stockage des pistons, avant leur assemblage dans les ensembles de seringues. Ce stockage a généralement lieu en disposant une pluralité de pistons dans un emballage commun. Si pendant cette phase de stockage des pistons se collent les uns aux autres, il peut s'avérer difficile de réaliser l'assemblage des ensembles de seringues sur les chaines de montage et d'assemblage. Il est donc souhaitable de pouvoir éviter tout risque de collage des pistons entre eux avant leur assemblage dans les ensembles de seringues, par insertion d'un piston respectif dans un réservoir respectif.

Selon une première variante des moyens anti-collage, représentée notamment sur la figure 8, la surface externe de ladite paroi d'extrémité proximale 24 du piston 20, qui est la surface du piston qui est en contact avec le produit fluide, comporte une zone annulaire 240 dont la surface est modifiée, notamment par grainage. Ce grainage peut être réalisé d'une quelconque manière appropriée, par exemple par modification physique du moule au niveau de ladite surface externe de ladite paroi d'extrémité proximale 24 du piston 20, notamment par électroérosion ou par projection de médias abrasifs. Ladite zone annulaire modifiée 240 n'inclut pas la pointe 25 de ladite paroi d'extrémité proximale 24 du piston 20 qui, après actionnement, coopère de manière étanche avec ladite ouverture de passage 15 de ladite paroi d'extrémité proximale 14 du réservoir 10. La zone annulaire modifiée 240 n'inclut pas non plus le bord radialement externe de ladite paroi d'extrémité proximale 14 du réservoir 10, qui coopère de manière étanche avec ladite paroi latérale cylindrique 11 du réservoir 10. Cette zone annulaire 240 permet d'éviter les risques de collage des pistons entre eux lors du stockage desdits pistons avant assemblage dans l'ensemble de seringue. En effet, comme visible sur la figure 8, la présence de la zone annulaire 240 va éviter l'effet ventouse qui pourrait se produire lorsque deux pistons se collent l'un à l'autre, avec la paroi d'extrémité proximale 24 d'un piston qui se colle de manière étanche contre l'extrémité distale 22 d'un autre piston. La zone annulaire 240 de surface modifiée empêche toute étanchéité à ce niveau, et donc tout risque de collage des pistons entre eux. Contrairement aux profils saillants de l'état de la technique, qui empêcheraient une fermeture étanche de l'ouverture de passage 15 du réservoir 10 par la pointe axiale 25 du piston 20 après actionnement, la présence de la zone annulaire 240 n'impacte pas ladite coopération étanche, malgré la faible différence entre les angles α et β. Alors que des profils saillants nécessiteraient une différence d'angle d'au moins 6°, voire 10°, comme dans les documents de l'état de la technique précités, ce qui augmente de manière négative le volume mort, la zone annulaire 240 permet d'éviter le collage de deux pistons lors du stockage, tout en minimisant ledit volume mort avec une très faible différence d'angle.

Selon une seconde variante des moyens anti-collage, représentée sur la figure 7, ladite extrémité distale 22 du piston 20 comporte une surface d'extrémité distale pourvue d'au moins un profil axialement saillant 220. Sur les figures, notamment sur la figure 4, il est représenté trois profils axialement saillants 220. Ces profils axialement saillants 220 permettent aussi d'éviter tout risque de collage entre deux pistons, comme représenté sur la figure 7. La présence desdits profils axialement saillants 220 empêche toute coopération étanche entre la paroi d'extrémité proximale 24 d'un piston et l'extrémité distale 22 d'un autre piston.

Avantageusement, le piston 20 peut comporter à la fois une zone annulaire 240 sur sa paroi d'extrémité proximale 24 et des profils axialement saillants sur son extrémité distale 22.

Bien que la présente invention ait été décrite en référence à des modes de réalisation avantageux de celle-ci, il est entendu que l'invention n'est pas limitée à ces modes de réalisation, mais qu'au contraire toutes modifications utiles peuvent être apportées par l'homme du métier sans sortir du cadre de la présente invention tel que défini par les revendications annexées.

## Revendications

1. Ensemble de seringue anti-reflux comprenant:
- un réservoir (10) destiné à contenir un produit fluide, ledit réservoir comportant une paroi latérale cylindrique (11), une extrémité distale (12) ouverte et une extrémité proximale (13) comportant une paroi d'extrémité proximale (14) pourvue en son centre axial d'une ouverture de passage (15),
- un piston (20) coulissant de manière étanche dans ledit réservoir (10), ledit piston (20) comportant une extrémité distale (22) adaptée à se connecter à une tige de piston (30) traversant ladite extrémité distale ouverte (12) du réservoir (10), et une extrémité proximale (23) comportant une paroi d'extrémité proximale (24),
**caractérisé en ce que** lesdites parois d'extrémités proximales (14, 24) du réservoir (10) et du piston (20) sont coniques, ladite paroi d'extrémité proximale (14) du réservoir (10) formant un angle α et ladite paroi d'extrémité proximale (24) du piston (20) formant un angle β, avec 3° < α - β < 5°, et **en ce que** la surface externe de ladite paroi d'extrémité proximale (24) du piston (20), en contact avec le produit fluide, comporte une zone annulaire (240) dont la surface est modifiée, notamment par grainage.

2. Ensemble selon la revendication 1, dans lequel α - β = 4°.

3. Ensemble selon la revendication 1 ou 2, dans lequel α = 145° (+/- 1°) et β = 141° (+/- 1°).

4. Ensemble selon l'une quelconque des revendications précédentes, dans lequel ladite zone annulaire modifiée (240) n'inclut pas la pointe (25) de ladite paroi d'extrémité proximale (24) du piston (20) qui, après actionnement, coopère de manière étanche avec ladite ouverture de passage (15) de ladite paroi d'extrémité proximale (14) du réservoir (10).

5. Ensemble selon l'une quelconque des revendications précédentes, dans lequel l'extrémité distale (22) du piston (20) comporte une surface d'extrémité distale pourvue d'au moins un profil axialement saillant (220).

6. Ensemble selon l'une quelconque des revendications précédentes, dans lequel ledit piston (20) comporte un pas de vis interne (27) adapté à se visser sur un pas de vis externe correspondant prévu sur ladite tige de piston (30), ledit piston (20) étant vissé sur ladite tige de piston (30).

## Patentansprüche

1. Antireflux-Spritzenanordnung, umfassend:
- einen Behälter (10) zur Aufnahme eines fluiden Produkts, wobei der Behälter eine zylindrische Seitenwand (11), ein offenes distales Ende (12) und ein proximales Ende (13) mit einer proximalen Endwand (14) aufweist, die in ihrer axialen Mitte mit einer Durchgangsöffnung (15) versehen ist,
- einen Kolben (20), der dicht in dem Behälter (10) gleitet, wobei der Kolben (20) ein distales Ende (22) aufweist, das zur Verbindung mit einer Kolbenstange (30) geeignet ist, die das offene distale Ende (12) des Behälters (10) durchquert, und ein proximales Ende (23) mit einer proximalen Endwand (24) aufweist,
**dadurch gekennzeichnet, dass** die proximalen Endwände (14, 24) des Behälters (10) und des Kolbens (20) konisch sind, wobei die proximale Endwand (14) des Behälters (10) einen Winkel a und die proximale Endwand (24) des Kolbens (20) einen Winkel β bildet, mit 3° < α - β < 5°, und dass die äußere Fläche der proximalen Endwand (24) des Kolbens (20) im Kontakt mit dem fluiden Produkt einen ringförmigen Bereich (240) aufweist, dessen Oberfläche modifiziert ist, insbesondere durch Aufrauhung.

2. Anordnung nach Anspruch 1, wobei a - β = 4° ist.

3. Anordnung nach Anspruch 1 oder 2, wobei α = 145° (+/- 1°) und β = 141° (+/- 1°) ist.

4. Anordnung nach einem der vorstehenden Ansprüche, wobei der modifizierte ringförmige Bereich (240) nicht die Spitze (25) der proximalen Endwand (24) des Kolbens (20) umfasst, die nach der Betätigung dicht mit der Durchgangsöffnung (15) der proximalen Endwand (14) des Behälters (10) zusammenwirkt.

5. Anordnung nach einem der vorstehenden Ansprüche, wobei das distale Ende (22) des Kolbens (20) eine distale Endfläche aufweist, die mit mindestens einem axial vorstehenden Profil (220) versehen ist.

6. Anordnung nach einem der vorstehenden Ansprüche, wobei der Kolben (20) ein Innengewinde (27) aufweist, das zum Aufschrauben auf ein entsprechendes Außengewinde an der Kolbenstange (30) geeignet ist, wobei der Kolben (20) auf die Kolbenstange (30) aufgeschraubt ist.

## Claims

1. An anti-reflux syringe assembly comprising:
- a reservoir (10) for containing a fluid, said reservoir having a cylindrical side wall (11), an open distal end (12), and a proximal end (13) having a proximal end wall (14) provided at its axial center with a through opening (15),
- a piston (20) slidable in leaktight manner in said reservoir (10), said piston (20) having a distal end (22) adapted to connect to a plunger (30) passing through said open distal end (12) of the reservoir (10), and a proximal end (23) having a proximal end wall (24),
**characterized in that** said proximal end walls (14, 24) of the reservoir (10) and of the piston (20) are conical, said proximal end wall (14) of the reservoir (10) forming an angle α and said proximal end wall (24) of the piston (20) forming an angle β, with 3° < α - β < 5°, and **in that** the outside surface of said proximal end wall (24) of the piston (20) in contact with the fluid has an annular zone (240) of surface that is modified, in particular by graining.

2. An assembly according to claim 1, wherein α - β = 4°.

3. An assembly according to claim 1 or claim 2, wherein α = 145° (+/- 1°) and β = 141° (+/- 1°).

4. An assembly according to any preceding claim, wherein said modified annular zone (240) does not include the tip (25) of said proximal end wall (24) of the piston (20), which tip, after actuation, co-operates in leaktight manner with said through opening (15) in said proximal end wall (14) of the reservoir (10).

5. An assembly according to any preceding claim, wherein the distal end (22) of the piston (20) has a distal end surface provided with at least one axially projecting profile (220).

6. An assembly according to any preceding claim, wherein said piston (20) has an inside screw thread (27) adapted to screw onto a corresponding outside screw thread provided on said plunger (30), said piston (20) being screwed on said plunger (30).
